(19) Europäisches Patentamt
European Patent Office
Office européen des brevets

(11) Publication number : **0 492 904 A1**

(12) **EUROPEAN PATENT APPLICATION**

(21) Application number : **91311531.7**

(22) Date of filing : **11.12.91**

(51) Int. Cl.[5] : **A61K 31/44,** A61K 31/55, C07D 453/02, C07D 471/08, C07D 487/08, // (C07D471/08, 221:00, 209:00), (C07D471/08, 223:00, 221:00), (C07D487/08, 209:00, 209:00)

(30) Priority : **21.12.90 GB 9027870**
**07.06.91 GB 9112308**

(43) Date of publication of application :
**01.07.92 Bulletin 92/27**

(84) Designated Contracting States :
**CH DE FR GB IT LI NL**

(71) Applicant : **MERCK SHARP & DOHME LTD.**
**Hertford Road**
**Hoddesdon Hertfordshire EN11 9BU (GB)**

(72) Inventor : **Lotti, Victor**
**214 Brookside Circle**
**Harleysville, Pennsylvania 19438 (US)**
Inventor : **Showell, Graham A.**
**5 Beehive Lane**
**Welwyn Garden City, Hertfordshire (US)**

(74) Representative : **Thompson, John Dr. et al**
**Merck & Co., Inc. European Patent**
**Department Terlings Park Eastwick Road**
**Harlow, Essex CM20 2QR (GB)**

# (54) Substituted benzene derivatives for use in the treatment of glaucoma.

(57) The use of a compound of structural formula (I) :

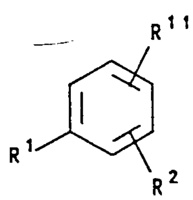

( I )

or a pharmaceutically acceptable salt thereof or a prodrug thereof ; wherein
$R^1$ represents a non-aromatic, non-fused 1-azabicyclic ring system selected from :

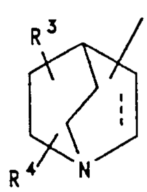

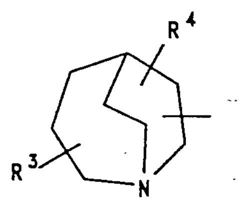

EP 0 492 904 A1

Jouve, 18, rue Saint-Denis, 75001 PARIS

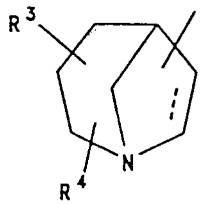

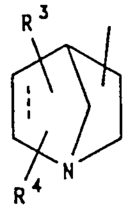

wherein the broken line represents an optional chemical bond ;

the substituents $R^3$ and $R^4$ may be present at any position, including the point of attachment to the benzene ring, and independently represent hydrogen, $C_{1-4}$ alkyl, halo, $C_{1-4}$ alkoxy, hydroxy, carboxy or $C_{1-4}$ alkoxycarbonyl, or $R^3$ and $R^4$ together represent carbonyl ;

and

$R^2$ and $R^{11}$ independently represent hydrogen, halo, $-CF_3$, $-OR^6$, $-NR^6R^7$, -CN, $-COR^8$, $C_{1-8}$ alkyl or $C_{2-8}$ alkenyl, provided that at least one of $R^2$ and $R^{11}$ is other than hydrogen, $C_{1-8}$ alkyl or $C_{2-8}$ alkenyl, or $R^2$ and $R^{11}$ taken together form a $C_{1-6}$ alkylenedioxy ring, wherein $R^6$ is $C_{1-6}$ alkyl, $R^7$ is hydrogen or $C_{1-6}$ alkyl, and $R^8$ represents -OH, $-OR^6$, $-NHR^7$ or $-NR^6R^7$ ;

for the manufacture of a medicament for treating glaucoma and/or for reducing intraocular pressure.

The present invention relates to a class of substituted benzene compounds for use in the treatment of glaucoma, formulations containing them and their synthesis.

Glaucoma is an ocular disorder associated with elevated intraocular pressures which are too high for normal function and may result in irreversible loss of visual function. If untreated, glaucoma may eventually lead to blindness. Ocular hypertension, i.e. the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Topical administration to the eye of agents such as pilocarpine may be used in order to improve the outflow of aqueous humour and reduce the intraocular pressure (see Drugs, 1979, vol. 17, 38; and Drugs and Ther. Bull., 1989, vol. 21, 85), but these have associated side effects such as emesis and myosis.

We have now found compounds which lower the intraocular pressure without exhibiting side effects to the extent associated with hitherto-known drugs used against glaucoma which act through cholinergic mechanisms.

In J. Med. Chem. (1981), 24 (12), 1475-82 is disclosed 3-hydroxy-3-(3-methoxyphenyl)quinuclidine and in Helv. Chim. Acta (1957), 40, 2170 is disclosed 3-(4-methoxyphenyl)quinuclidine. No therapeutic activity is ascribed to either compound. In Khim.-Farm. Zh. (1982), 16 (3), 307-311 are disclosed 3-hydroxy-3-(4-methoxyphenyl)quinuclidine, 3-hydroxy-3-(2-methoxyphenyl)quinuclidine and 3-(4-chlorophenyl)-3-hydroxyquinuclidine; the authors describe how these compounds did not display pharmacological activity. Indeed, as reported in the J. Med. Chem. article mentioned above, related compounds were tested for dopaminergic activity and found to be essentially inactive.

It was therefore unexpected to find that a class of benzene compounds do exhibit therapeutic activity and have been found to reduce intraocular pressure without exhibiting severe adverse effects on pupil size.

The compounds for use according to the present invention may be represented by structural formula (I):

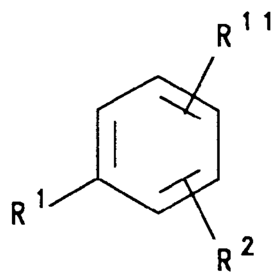

( I )

or a salt or prodrug thereof; wherein

$R^1$ represents a non-aromatic, non-fused 1-azabicyclic ring system selected from:

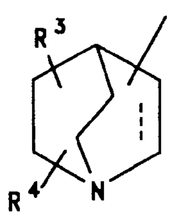

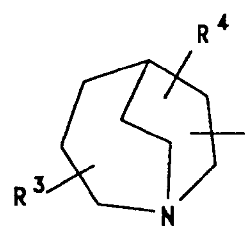

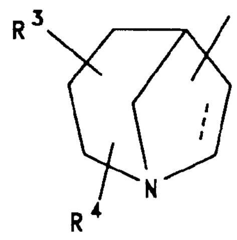 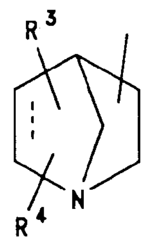

wherein the broken line represents an optional chemical bond;

the substituents $R^3$ and $R^4$ may be present at any position, including the point of attachment to the benzene ring, and independently represent hydrogen, $C_{1-4}$ alkyl, halo, $C_{1-4}$ alkoxy, hydroxy, carboxy or $C_{1-4}$ alkoxycarbonyl, or $R^3$ and $R^4$ together represent carbonyl; and

$R^2$ and $R^{11}$ independently represent hydrogen, halo, $-CF_3$, $-OR^6$, $-NR^6R^7$, -CN, $-COR^8$, $C_{1-8}$ alkyl or $C_{2-8}$ alkenyl, provided that at least one of $R^2$ and $R^{11}$ is other than hydrogen, $C_{1-8}$ alkyl or $C_{2-8}$ alkenyl, or $R^2$ and $R^{11}$ taken together form a $C_{1-6}$ alkylenedioxy ring, wherein $R^6$ is $C_{1-6}$ alkyl, $R^7$ is hydrogen or $C_{1-6}$ alkyl, and $R^8$ represents -OH, $-OR^6$, $-NHR^7$ or $-NR^6R^7$.

In the definition of $R^1$, it will be appreciated that the nitrogen atom in the azabicyclic ring system will carry a lone pair of electrons.

In the definition of $R^2$ and/or $R^{11}$, unless otherwise specified, "alkyl" and "alkenyl" groups may be straight, branched or cyclic groups.

In formula (I), preferably, $R^2$ and $R^{11}$ are each independently selected from hydrogen, halo (F, Cl, Br, I), $-CF_3$ and $-OR^6$, provided that one of $R^2$ and $R^{11}$ is other than hydrogen, or $R^2$ and $R^{11}$ taken together form a $C_{1-6}$ alkylenedioxy ring.

Suitably, the group $R^3$ is hydrogen or methyl; and $R^4$ is hydrogen, $C_{1-4}$ alkoxy, $C_{1-4}$ alkyl, $C_{1-4}$ alkoxycarbonyl, halo or hydroxy; preferably methoxy, methyl, fluoro, chloro, hydroxy or methoxycarbonyl. Preferably, one or both of $R^3$ and $R^4$ is hydrogen. More preferably, $R^4$ is hydroxy or hydrogen when $R^3$ is hydrogen.

Suitably, $R^1$ is azanorbornane, quinuclidine, 1-azabicyclo[2.2.2]octene (dehydroquinuclidine) or 1-azabicyclo[3.2.1]octane, any of which may in particular be either unsubstituted or substituted with methyl, hydroxy, fluoro, chloro or methoxycarbonyl. Preferably, $R^1$ is quinuclidine, 2,3-(dehydro)quinuclidine, 1-azabicyclo[2.2.1]heptane or 1-azabicyclo[3.2.1]octane, especially quinuclidine or 2,3-(dehydro)quinuclidine, optionally substituted with hydroxy.

A particularly preferred subclass of compounds of formula (I) or salts or prodrugs thereof are those wherein:

$R^1$ is a ring system shown above, optionally substituted by hydroxy; and

$R^2$ and $R^{11}$ are each independently selected from hydrogen, halo (especially chloro), $-CF_3$ and $-OR^6$ (especially $-OCH_3$), provided that at least one of $R^2$ and $R^{11}$ is other than hydrogen, or $R^2$ and $R^{11}$ taken together form a $C_{1-6}$ alkylenedioxy ring (especially 3,4-methylenedioxy or 3,4-ethylenedioxy).

Especially preferred is wherein:

$R^1$ is 1-azabicyclo[2.2.2]octyl, 2,3-dehydro-1-azabicyclo[2.2.2]octyl, 1-azabicyclo[3.2.1]octyl, 2,3-dehydro-1-azabicyclo[3.2.1]octyl, 1-azabicyclo[2.2.1]-heptyl or 2,3-dehydro-1-azabicyclo[2.2.1]heptyl, any of which may be optionally substituted by hydroxy.

For example, $R^1$ may be 1-azabicyclo[2.2.1]heptyl or quinuclidinyl, preferably quinuclidinyl;

$R^2$ may be methoxy or halo, preferably methoxy or fluoro;

$R^{11}$ may be hydrogen, methoxy or halo, preferably hydrogen, methoxy or fluoro; or

$R^2$ and $R^{11}$ taken together may form ethylenedioxy; and

$R^3$ and $R^4$ may each be hydrogen.

The present invention includes within its scope prodrugs of the compounds of formula I above. In general, such prodrugs will be functional derivatives of the compounds of formula I which are readily convertible in vivo into the required compound of formula I. Conventional procedures for the selection and preparation of suitable prodrug derivatives are described, for example, in "Design of Prodrugs", ed. H. Bundgaard, Elsevier, 1985.

Most of the compounds for use according to this invention have at least one asymmetric centre and often more than one; and can therefore exist as both enantiomers and diastereoisomers. In particular, those compounds possessing an unsymmetrical azabicyclic ring system may exist as exo and endo diastereoisomers. It is to be understood that the invention covers the use of all such isomers and mixtures thereof.

Also included within the scope of the present invention is the use of salts of the compounds of formula (I). It will be appreciated that salts of the compounds for use in medicine will be non-toxic pharmaceutically acceptable salts. Other salts may, however, be useful in the preparation of the compounds of use in the invention or their non-toxic pharmaceutically acceptable salts. Acid addition salts, for example, may be formed by mixing a solution of the compound with a solution of a pharmaceutically acceptable non-toxic acid such as hydrochloric acid, fumaric acid, oxalic acid, maleic acid, succinic acid, acetic acid, citric acid, tartaric acid, p-toluenesulphonic acid, carbonic acid or phosphoric acid. Preferred are the hydrochloride, hydrogen oxalate, maleate, tartrate and tosylate salts. Where the compound carries a carboxylic acid group the invention also contemplates use of salts thereof, preferably non-toxic pharmaceutically acceptable salts thereof, such as the sodium, potassium and calcium salts thereof.

Salts of amine groups may also comprise the quaternary ammonium salts in which the amino nitrogen atom carries an alkyl, alkenyl, alkynyl or aralkyl group.

Examples of compounds for use according to this invention include:
3-(2,4-dimethoxyphenyl)quinuclidine;
3-(2,5-dimethoxyphenyl)quinuclidine;
3-(3,4-ethylenedioxyphenyl)quinuclidine;
endo-3-(3-methoxyphenyl)-1-azabicyclo[2.2.1]heptane;
and salts and prodrugs thereof.

In addition, the following compounds [each of which is referred to hereinafter as a "previously undisclosed compound of formula (I)"] are not specifically disclosed in the prior art. They are accordingly novel compounds, and represent a further feature of the present invention:
3-(2-methoxyphenyl)quinuclidine;
3-(3-fluorophenyl)quinuclidine;
3-(3,5-difluorophenyl)quinuclidine;
and salts and prodrugs thereof.

The present invention also provides a pharmaceutical composition comprising a "previously undisclosed compound of formula (I)" as defined above or a pharmaceutically acceptable salt thereof or a prodrug thereof in association with one or more pharmaceutically acceptable carriers and/or excipients.

The present invention further provides a "previously undisclosed compound of formula (I)" as defined above or a pharmaceutically acceptable salt thereof or a prodrug thereof for use in therapy.

In a still further aspect, the present invention provides the use of a "previously undisclosed compound of formula (I)" as defined above or a pharmaceutically acceptable salt thereof or a prodrug thereof for the manufacture of a medicament for treating glaucoma and/or for reducing intraocular pressure.

The compounds of formula (I) above, including the novel compounds according to this invention, may be prepared as described hereinbelow or by processes analogous thereto known to persons skilled in the art.

The compounds of formula (I) above, including the novel compounds according to this invention, wherein $R^3$ and $R^4$ are each other than hydroxy- or carboxy-substituted on the azabicycle at the point of attachment to the benzene ring may be prepared by a process which comprises the dehydroxylation or decarboxylation of a compound of formula (III) [which is a sub-class of the compounds of formula (I)] or a salt thereof:

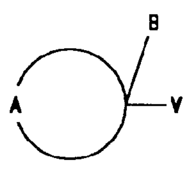

(III)

wherein V represents a benzene ring substituted by the groups $R^2$ and $R^{11}$ as defined above; A represents the residue of an azabicyclic ring as defined above in $R^1$; and B represents hydroxy or carboxy.

When the group B in compound (III) is hydroxy, it may be removed by chlorination and elimination or by dehydration to provide compounds of formula (I) wherein $R^1$ contains unsaturation at the point of attachment to the benzene ring. When it is desired to prepare a compound of formula (I) wherein $R^1$ contains saturation at the point of attachment to the benzene ring, the unsaturated compound of formula (I) may be hydrogenated using conventional methods. Dehydration may be accomplished by treatment with an acid, for example with trifluoroacetic acid. For example, chlorination and elimination may be effected by treatment with phosphorus oxychloride in the presence of triethylamine, or with thionyl chloride followed, where necessary, by DBN. The chloride or the unsaturated product may then be hydrogenated under conventional conditions, such as over

10% palladium/carbon in methanol. Alternatively, the compound (III) may be dehydroxylated by the use of thionyl chloride followed by treatment with tributyltin hydride in a solvent such as tetrahydrofuran in the presence of a radical initiator such as azabisisobutyronitrile.

The compound of formula (III) where B is hydroxy may be prepared by reaction of a ketone compound of formula (IV) with a metal derivative of a benzene compound of formula (V):

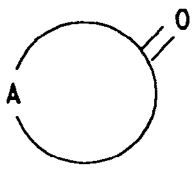

(IV)

$$M - V$$

(V)

wherein A and V are as defined above; and M represents a metal atom, for example magnesium. The metal derivative for instance may be prepared by reacting the corresponding halo-substituted benzene compound such as the Br- or I-substituted benzene compound with the metal.

When the group B in compound (III) is carboxy it may be removed by standard decarboxylation techniques such as heating in aqueous solution made to pH1 with hydrochloric acid.

The compounds of formula (III) where B represents carboxy may be prepared by reaction of a compound of formula (VI) with a compound of formula (VII):

$$R^1 - W$$

(VI)

$$Hal - V$$

(VII)

wherein $R^1$ and V are as defined above, Hal represents halo, and W represents cyano, a carboxylic acid group or a derivative thereof which activates the adjacent position; and subsequently, where necessary, converting the group W to carboxy, preferably by hydrolysis.

Preferably, W represents an alkyl ester group such as methoxycarbonyl. Preferably, the halo group is iodo. The reaction between compounds (VI) and (VII) may be carried out in the presence of a strong base such as lithium diisopropylamide in a solvent such as tetrahydrofuran.

The azabicyclic moiety may be introduced into the molecules concerned by methods known from the art, in particular by methods analogous to those described in EP-A-0239309.

After any of the above described processes is complete, one substituent can be converted to another. For example, an amino group may be converted to chloro, or hydrazo, $-NHNH_2$, via the intermediacy of diazonium, $-N_2$. Similarly, alkoxycarbonyl groups may be converted, via carboxy, to an amino substituent, $-NH_2$; and methoxy may be converted to hydroxy by treatment with concentrated hydrobromic acid; these groups may then be further converted to any value of $R^2$ defined above.

In any of the above reactions it may be necessary and/or desirable to protect any sensitive groups in the compounds. For example, if the reactants employed include amino, carboxy, keto, hydroxy or thiol groups, these may be protected in conventional manner. Thus, suitable protecting groups for hydroxy groups include silyl groups such as trimethylsilyl or t-butyl-dimethylsilyl, and etherifying groups such as tetrahydropyranyl; and for amino groups include benzyloxycarbonyl and t-butoxycarbonyl. Keto groups may be protected in the form of a ketal. Carboxy groups are preferably protected in a reduced form such as in the form of their corresponding protected alcohols, which may be subsequently oxidised to give the desired carboxy group. Thiol groups may be protected by disulphide formation, either with the thiol itself or with another thiol to form a mixed disulphide. The protecting groups may be removed at any convenient stage in the synthesis of the desired compound according to conventional techniques.

When administered for the treatment of elevated intraocular pressure or glaucoma, the active compound is preferably administered topically to the eye, although systemic treatment is also possible. The dose administered can be from as little as 0.1 to 25 mg or more per day, singly, or preferably on a 2 to 4 dose per day regimen although a single dose per day is satisfactory.

The present invention therefore also provides a pharmaceutical formulation suitable for use in reducing

intraocular pressure or for treating glaucoma which formulation comprises a compound of formula (I) and a pharmaceutically acceptable carrier.

It will be understood that any formulation may further comprise another active ingredient such as another antiglaucoma agent for example a topical carbonic anhydrase inhibitor.

When given systemically, the drug can be given by any route, although the oral route is preferred. In oral administration, the drug can be employed in any of the usual dosage forms such as tablets or capsules, either in a contemporaneous delivery or sustained release form. Any number of the usual excipients or tabletting aids can likewise be included.

When given by the topical route, the active compound or an ophthalmologically acceptable salt thereof such as the hydrochloride salt is formulated into an ophthalmic preparation. In such formulations, from 0.0005% to 15% by weight can be employed. The objective is to administer a dose of from 0.1 to 1.0 mg per eye per day to the patient, with treatment continuing so long as the condition persists.

Thus, in an ophthalmic solution, insert, ointment or suspension for topical delivery, or a tablet, intramuscular or intravenous composition for systemic delivery, the active medicament or an equivalent amount of a salt thereof is employed, the remainder being carrier, excipients, preservatives and the like as are customarily used in such compositions.

The active drugs for use according to this invention are most suitably administered in the form of ophthalmic pharmaceutical compositions adapted for topical administration to the eye such as a suspension, ointment, or as a solid insert. A preferred composition is eye drops. Formulations of these compounds may contain from 0.0005 to 15% and especially 0.05% to 2% of medicament. Higher dosages as, for example, about 10% or lower dosages can be employed provided the dose is effective in reducing or controlling elevated intraocular pressure. As a unit dosage from between 0.001 to 10.0 mg, preferably 0.005 to 2.0 mg, and especially 0.1 to 1.0 mg of the compound is generally applied to the human eye, generally on a daily basis in single or divided doses so long as the condition being treated persists.

This invention therefore further provides a pharmaceutical formulation adapted for topical administration to the eye which formulation comprises a compound of formula (I) and a carrier suitable for topical administration.

The hereinbefore-described dosage values are believed accurate for human patients and are based on the known and presently understood pharmacology of the compounds, and the action of other similar entities in the human eye. As with all medications, dosage requirements are variable and must be individualized on the basis of the disease and the response of the patient.

For topical administration, pharmaceutically acceptable carriers are, for example, water, mixtures of water and water-miscible solvents such as lower alkanols or arylalkanols, vegetable oils, polyalkylene glycols, petroleum based jelly, ethyl cellulose, ethyl oleate, carboxymethylcellulose, polyvinylpyrrolidone, isopropyl myristate and other conventionally-employed non-toxic, pharmaceutically acceptable organic and inorganic carriers. The pharmaceutical preparation may also contain non-toxic auxiliary substances such as emulsifying, preserving, wetting and bodying agents, for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenyl ethanol, buffering ingredients such as sodium chloride, sodium borate, sodium acetates, gluconate buffers, and other conventional ingredients such as sorbitan monolaurate, triethanolamine oleate, polyoxyethylene sorbitan monopalmitylate, dioctyl sodium sulfosuccinate, monothioglycerol, thiosorbitol and ethylenediamine tetraacetic acid. Additionally, suitable ophthalmic vehicles can be used as carrier media for the present purpose including conventional phosphate buffer vehicle systems, isotonic boric acid vehicles, isotonic sodium chloride vehicles and isotonic sodium borate vehicles.

The pharmaceutical formulation may also be in the form of a solid insert such as one which after dispensing the drug remains essentially intact, or a bio-erodible insert that is soluble in lachrymal fluids or otherwise disintegrates.

The present invention will now be illustrated by the following Descriptions and Examples.

## DESCRIPTION A

3-(2,4-Dimethoxyphenyl)quinuclidine Hydrogen Oxalate

Prepared as described in Example 19 of EP-A-0412797.

**DESCRIPTION B**

3-(2,5-Dimethoxyphenyl)quinuclidine Hydrogen Oxalate

Prepared as described in Example 20 of EP-A-0412797.

**DESCRIPTION C**

3-(3,4-Ethylenedioxyphenyl)quinuclidine Hydrogen Maleate

Prepared as described in Example 22 of EP-A-0412797.

**DESCRIPTION D**

endo-3-(3-Methoxyphenyl)-1-azabicyclo[2.2.1]heptane Hydrogen Tartrate

Prepared as described in Example 47 of EP-A-0412797.

**EXAMPLE 1**

3-(2-Methoxyphenyl)quinuclidine Hydrogen Oxalate

Prepared from 3-quinuclidinone and 2-methoxybromobenzene in a manner similar to Description A. The hydrogen oxalate salt had m.p. 165-167°C. (Found: C, 61.86; H, 7.07; N, 4.42. $C_{14}H_{19}NO$. 1.1 $C_2H_2O_4$ requires C, 61.50; H, 6.75; N, 4.43%).

**EXAMPLE 2**

3-(3-Fluorophenyl)quinuclidine Hydrogen Tartrate

Prepared from 3-quinuclidinone and 3-fluorobromobenzene in a manner similar to Description A. The hydrogen tartrate salt had m.p. 128-129°C. (Found: C, 57.14; H, 5.94; N, 3.78; F, 5.83. $C_{13}H_{16}FN.C_4H_6O_6$ requires C, 57.46; H, 6.24; N, 3.94; F, 5.35%).

**EXAMPLE 3**

3-(3,5-Difluorophenyl)quinuclidine Hydrogen Tartrate

Prepared from 3-quinuclidinone and 3,5-difluorobromobenzene in a manner similar to Description A. The hydrogen oxalate salt had m.p. 126-130°C. (Found: C, 53.96; H, 5.41; N, 3.69; F, 9.09. $C_{13}H_{15}F_2N$. 1.1 $C_4H_6O_6$ requires C, 53.81; H, 5.60; N, 3.61; F, 9.78%).

## BIOLOGICAL ACTIVITY

| Compound of | Concentration | Change in IOP[a] |
|---|---|---|
| Description A | 0.05% | -2.0 |
| Description B | 0.05% | -2.0 |
| Description C | 0.5% | -3.0 |
| Description D | 0.5% | -2.0 |
| Example 1 | 0.5% | -1.5 |
| Example 2 | 0.5% | -4.0 |
| Example 3 | 0.5% | -3.5 |

FOOTNOTE: a, Maximum mmHg change in intraocular pressure

## FORMULATIONS

The compounds of the Descriptions and Examples may be formulated as follows:

### Eye Drops

| | |
|---|---|
| The pharmaceutically acceptable salt of the active compound | 0.5% |
| Benzalkonium chloride solution | 0.02% v/v |
| Disodium edetate | 0.05% |
| NaCl | 0.8% |
| Water for injections | to 100% |

The formulation is sterilised by autoclaving.

## Claims

1. The use of a compound of structural formula (I):

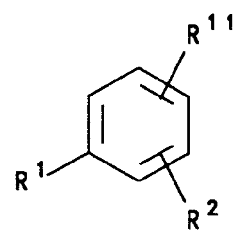

(I)

or a pharmaceutically acceptable salt thereof or a prodrug thereof; wherein

$R^1$ represents a non-aromatic, non-fused 1-azabicyclic ring system selected from:

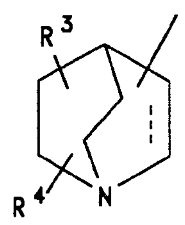

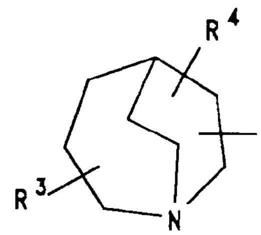

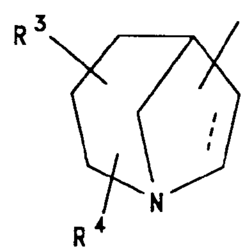

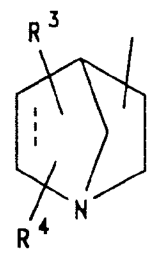

wherein the broken line represents an optional chemical bond;

the substituents $R^3$ and $R^4$ may be present at any position, including the point of attachment to the benzene ring, and independently represent hydrogen, $C_{1-4}$ alkyl, halo, $C_{1-4}$ alkoxy, hydroxy, carboxy or $C_{1-4}$ alkoxycarbonyl, or $R^3$ and $R^4$ together represent carbonyl; and

$R^2$ and $R^{11}$ independently represent hydrogen, halo, $-CF_3$, $-OR^6$, $-NR^6R^7$, -CN, $-COR^8$, $C_{1-8}$ alkyl or $C_{2-8}$ alkenyl, provided that at least one of $R^2$ and $R^{11}$ is other than hydrogen, $C_{1-8}$ alkyl or $C_{2-8}$ alkenyl, or $R^2$ and $R^{11}$ taken together form a $C_{1-6}$ alkylenedioxy ring, wherein $R^6$ is $C_{1-6}$ alkyl, $R^7$ is hydrogen or $C_{1-6}$ alkyl, and $R^8$ represents -OH, $-OR^6$, $-NHR^7$ or $-NR^6R^7$;

for the manufacture of a medicament for treating glaucoma and/or for reducing intraocular pressure.

2. The use as claimed in claim 1 wherein

$R^1$ is as defined in claim 1;

one of $R^3$ and $R^4$ represents hydrogen and the other represents hydrogen or hydroxy; and

$R^2$ and $R^{11}$ independently represent hydrogen, halo, trifluoromethyl or $C_{1-6}$ alkoxy, provided that at least one of $R^2$ and $R^{11}$ is other than hydrogen, or $R^2$ and $R^{11}$ taken together form a $C_{1-6}$ alkylenedioxy ring.

3. The use as claimed in claim 1 or claim 2 wherein $R^1$ is 1-azabicyclo[2.2.2]octyl, 2,3-dehydro-1-azabicyclo[2.2.2]octyl, 1-azabicyclo[3.2.1]octyl, 2,3-dehydro-1-azabicyclo[3.2.1]octyl, 1-azabicyclo[2.2.1]heptyl or 2,3-dehydro-1-azabicyclo[2.2.1]heptyl, any of which may be optionally substituted by hydroxy.

4. The use as claimed in any one of the preceding claims wherein

$R^1$ is 1-azabicyclo[2.2.1]heptyl or quinuclidinyl;

$R^2$ is methoxy or halo, and $R^{11}$ is hydrogen, methoxy or halo; or $R^2$ and $R^{11}$ taken together are ethylenedioxy; and

$R^3$ and $R^4$ each represents hydrogen.

5. The use as claimed in any one of the preceding claims of a compound selected from:

3-(2,4-dimethoxyphenyl)quinuclidine;

3-(2,5-dimethoxyphenyl)quinuclidine;

3-(3,4-ethylenedioxyphenyl)quinuclidine;

endo-3-(3-methoxyphenyl)-1-azabicyclo[2.2.1]heptane;

and pharmaceutically acceptable salts thereof and prodrugs thereof.

6. A compound selected from:

3-(2-methoxyphenyl)quinuclidine;
3-(3-fluorophenyl)quinuclidine;
3-(3,5-difluorophenyl)quinuclidine;
and salts and prodrugs thereof.

**7.** A pharmaceutical composition comprising a compound as claimed in claim 6 or a pharmaceutically acceptable salt thereof or a prodrug thereof in association with one or more pharmaceutically acceptable carriers and/or excipients.

**8.** The use of a compound as claimed in claim 6 or a pharmaceutically acceptable salt thereof or a prodrug thereof for the manufacture of a medicament for treating glaucoma and/or for reducing intraocular pressure.

**9.** A pharmaceutical composition adapted for use in reducing intraocular pressure and/or for treating glaucoma which comprises a compound of formula (I) as defined in claim 1 or a pharmaceutically acceptable salt thereof or a prodrug thereof in association with a pharmaceutically acceptable carrier.

**10.** A pharmaceutical composition adapted for topical administration to the eye which comprises a compound of formula (I) as defined in claim 1 or a pharmaceutically acceptable salt thereof or a prodrug thereof in association with a carrier suitable for topical administration.

European Patent Office

# EUROPEAN SEARCH REPORT

Application Number

EP 91 31 1531

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF NEUROCHEMISTRY<br>vol. 46, no. 4, 1986,<br>pages 1170 - 1175;<br>G.H. STERLING ET AL: 'Inhibition of high-affinity choline uptake and acetylcholine synthesis by quinuclidinyl and hemicholinium derivatives'<br>* table 1, compound X; page 1174, column 1, line 53 - column 2, line 2 * | 1,9,10 | A61K31/44<br>A61K31/55<br>C07D453/02<br>C07D471/08<br>C07D487/08<br>//(C07D471/08, 221:00,209:00)<br>(C07D471/08, 223:00,221:00)<br>(C07D487/08, 209:00,209:00) |
| A | EP-A-0 370 415 (BOEHRINGER INGELHEIM KG)<br>* page 3, line 42 - line 48; claims 1,3-5 * | 1 | |
| A | GB-A-865 974 (CIBA LTD)<br>* page 1, line 9 - line 29; examples 3,4 * | 1,9,10 | |
| A | GB-A-865 973 (CIBA LTD)<br>* page 1, line 7 - line 25; example 2 * | 1,9,10 | |
| P,A,D | EP-A-0 412 797 (MERCK SHARP & DOHME LTD)<br>* claims 1-8 * | 1-10 | |
| | | | TECHNICAL FIELDS SEARCHED (Int. Cl.5) |
| | | | A61K<br>C07D |

The present search report has been drawn up for all claims

| Place of search | Date of completion of the search | Examiner |
|---|---|---|
| BERLIN | 08 APRIL 1992 | van Amsterdam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)